# EUROPEAN PATENT SPECIFICATION

(11) **EP 1 432 457 B1**
(45) Date of publication and mention of the grant of the patent: **08.07.2009**
(21) Application number: 02763808.9
(22) Date of filing: 01.10.2002
(51) Int. Cl.: A61L 15/34, A61F 13/15

(54) **SANITARY NAPKINS WITH HYDROPHOBIC LOTIONS**
MONATSBINDEN MIT HYDROPHOBEN LOTIONEN
SERVIETTES HYGIENIQUES AVEC LOTIONS HYDROPHOBES

(30) Priority: 01.10.2001 US 968154; 21.05.2002 US 152924
(43) Date of publication of application: 30.06.2004
(73) Proprietor: THE PROCTER & GAMBLE COMPANY, Cincinnati, Ohio 45202 (US)
(72) Inventor: HAMMONS, John, Lee, Hamilton, OH 45011 (US); WARREN, Raphael, Cincinnati, OH 45237 (US); VISSCHER, Ronald, Bosman, Glendale, OH 45246 (US); GATTO, Joseph, Anthony, Loveland, OH 45140 (US); GRAY, Brian, Francis, Wyoming, OH 45215 (US)
(74) Representative: Briatore, Andrea
(86) International application number: PCT/US2002/031135
(87) International publication number: WO 2003/028776

(56) References cited:
- EP-A- 1 051 958
- WO-A-00/69485

## Description

### FIELD OF INVENTION

This application relates to catamenial devices such as sanitary napkins for the absorption of menses. More particularly, the present invention relates to catamenial devices having a hydrophobic lotion coating on the outer surface of the topsheet, the lotion being transferable to the wearer's skin by normal contact and wearer motion and/or body heat.

### BACKGROUND OF THE INVENTION

Disposable absorbent articles, such as diapers, training pants, and catamenial devices having lotioned topsheets are known. Lotions of various types are known to provide various skin benefits, such as prevention or treatment of diaper rash. These lotions can be applied to the topsheet of absorbent articles, for example, and can be transferred to the skin of the wearer during use.

Unlike many types of disposable absorbent articles, catamenial devices, such as pads and pantyliners are specifically designed to acquire menstrual fluid. Menstrual fluid differs from other exudates, such as urine, in many important properties, such as viscosity. Therefore, catamenial devices should differ in their structural components from such devices as baby diapers to be optimized for the maximum absorption of menstrual fluid.

The addition of lotion to the topsheet of absorbent articles is known to provide benefits such as easier BM clean up on babies. Likewise, lotion on topsheets is known to provide for better skin health of babies, such as the reduction of diaper rash. For example, U.S. Pat. No. 3,489,148 to Duncan et al. teaches a baby diaper comprising a discontinuous film of oleaginous material. A major disadvantage of the diapers disclosed in the Duncan et al. reference is that the hydrophobic and oleophobic topsheets are slow in promoting transfer of urine to the underlying absorbent cores. Since the viscosity of menses is considerably greater than urine, the problems associated with Duncan et al are more profound.

One successful attempt at overcoming the problems of Duncan is disclosed in Roe et al., U.S. Pat. No. 5,968,025. Roe et al. discloses an absorbent article in which a lotion is applied to a hydrophilic topsheet (or a topsheet rendered to be hydrophilic). The hydrophilic topsheet aids in ensuring urine gushes are adequately absorbed into the underlying core, rather than running off into the sides of a baby diaper, for example.

Other attempts in this respect are disclosed in EP-A-1051958 and WO-A-00/69485.

The known attempts at applying lotions to topsheets of absorbent products have been primarily directed to baby diapers, with the benefit provided being better skin health for the bottom of the baby. Little attention has been directed to the unique problems associated with the skin of an adult woman when wearing a catamenial pad. The skin of the vulvar area of an adult woman is very different than that of a baby's bottom (or buttock skin in general), and the lotion needs are very different. For example, rather than being concerned with diaper rash, a menstruating woman is more concerned about hygiene, that is, reducing the amount of menses remaining on the skin and hair after use of a sanitary pad.

The aforementioned attempts at providing a lotion on a topsheet of an absorbent article have focused on the loltion/topsheet characteristics necessary to handle a gush of urine in a relatively short amount of time. However, for catamenial devices, the fluid insult has very different characteristics, in the context of physio-chemical properties (e.g., viscosity, fluid dynamics, etc.) and in the volume and in the time to be absorbed. For example, menstrual flow typically consists of two patterns. One of these is "trickle" flow, which varies from 0.1 to 2 ml per hour. The second pattern is "gush" flow which varies from a few ml in volume delivered over a few seconds. Gush flow can result from an accumulation of menses pooling in the vagina which can then exit the body upon a change in position, such as a transition from sitting to standing. In any event, even with gush flow, the total amount of fluid required to be absorbed into the core in a given time is much less than that required by other absorbent produces, such as baby diapers, for example. One practical result is that catamenial devices, rather than needing to be deigned to handle gushing fluid, more typically handle fluid through a "blotting" effect.

Accordingly, there is a need for an improvement in catamenial devices to improve the skin hygiene of menstruating women.

Additionally, there is a need for a catamenial device having improved fluid handling such that more menses enter into and remain in the device, and less on the skin and hair of the wearer.

Further, then is a need for a catamenial device that that can change the skin/device interface properties when the device is worn.

### SUMMARY OF THE INVENTION

A catamenial device is disclosed, the device having a topsheet having a body facing surface, wherein the topsheet has a contact angle with water of 90 degrees or greater. A lotion composition as characterized in claim 1 is applied, to at least a portion of at leat to the body facing surface of the topsheet, the lotion composistion having a level of hydrophobicity equal or greater than that of said topsheet. A backsheet is joined to the topsheet and an absorbent core is disposed between the topsheet and said backsheet.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:
FIG. I is a perspective view of a catamenial device having a topsheet and a lotion composition.

### DETAILED DESCRIPTION OF THE INVENTION

FIG. 1 shows a catamenial device 10, that can be a sanitary napkin or pantyliner, having a body-contacting surface 12 comprising a topsheet 14, a liquid impervious backsheet 16 joined to the topsheet 14, an absorbent core 18. The sanitary napkin 10 has a longitudinal axis L and may also be provided with additional features commonly found in napkins, including "wings" or "flaps" (not shown) as is known in the art, and , and/or a fluid acquisition layer to promote fluid transport to the absorbent core 18. Likewise, the topsheet of the sanitary napkin can have various optional characteristics, as is known in the art. For example, the topsheet 14 can have channels embossed therein to direct fluid flow, and can have apertures therethrough to aid in fluid acquisition. The topsheet 14 of the catamenial device 10 of the present invention has a lotion composition 22 disposed onto the topsheet.

The topsheet 14 and lotion composition 22 of the present invention offer significant advantages over known topsheets and lotions. In particular, in a preferred embodiment, the topsheet 14 is hydrophobic or rendered to be hydrophobic, and the lotion is also hydrophobic. The levels of hydrohobicity can be determined by standard techniques, such as measuring angles that a drop of water make on a surface of material at equilibrium. In general, for the purposes of this invention, a material is considered hydrophobic if a drop of water exhibits an angle of 90 degrees or greater. Fibers are considered to be hydrophobic if film sheets formed from the polymers of the fibers would exhibit contact angles with water greater than 90 degrees. Contact angles as a measure of hydrophobicity are well known in the art, and methods for measuring contact angles are equally well known. As is well known, contact angles greater than 90 degrees are considered hydrophobic, and contact angles less than 90 degrees are considered hydrophilic.
The levels of hydrophobicity of the topsheet and lotion, respectively, can be equal, or the hydrophobicity of the lotion can be greater than the hydrophobicity of the topsheet. In use, the lotion can transfer from the topsheet to the skin of the wearer, which serves to make the skin and hair hydrophobic as well.

The advantage of the present invention can be appreciated with an understanding of the difference between menstrual fluid flow and urine flow in babies, for example. Topsheets of baby diapers are generally taught to be hydrophilic, with or without a lotion applied, such that sudden gushes of urine can be acquired through the topsheet and into the core with minimal runoff of fluid. However, it has been discovered that menstrual fluid, which has a much greater viscosity and much lower fluid flow, both in quantity and time, can be very effectively handled with a hydrophobic topsheet. Whereas urine may simply run off of a hydrophobic topsheet, particularly one that is heated with a hydrophobic lotion, it has unexpectedly been found that such a structure provides for superior benefits in a catamenial pad for menstruating women. Another unexpected benefit is the coating of the skin and hair of the vulvar region during use of a catamenial device of the present invention that results in cleaner skin and hair of the vulvar region. Yet, another benefit is better fluid acquisition of the fluid due to transfer of the lotion to the skin of the wearer that minimizes fluid transport on the skin and hair of the wearer away from the point of exit.

Without being bound by theory, it is believed that the superior benefits of the present invention are best exhibited by the combination of a hydrophobic topsheet and a hydrophobic lotion. A lotion is considered hydrophobic, for example, if the hydrophilic/lipophilic balance (HLB) is less than or equal to 7.

The lotion compositions of the present invention can comprise a select combination of skin treatment agents such as hexamidine, zinc oxide, and niacinamide which are highly effective in the prevention and treatment of erythema, malodor, and bacterial skin disorders, especially when these lotion compositions are administered to the skin from application on absorbent articles.

The term "skin treatment agent" as used, herein refers to materials that when applied topically and internally to the skin are capable of preventing, reducing, and/or eliminating any occurrence of skin disorders, particularly skin disorders associated with erythema, malodor, and bacterial infections. The term "skin disorders" as used herein refers to symptoms associated with irritating, acute, or chronic skin abnormalities. Examples of such symptoms include, but are not limited to, itching, inflammation, rash, burning, stinging, redness, swelling, sensitivity, sensation of heat, flaking/scaling, malodor, and the like. The term "ambient conditions" as used herein refers to surrounding conditions at about one atmosphere of pressure, at 50% relative humidity, and at 25°C.

The lotion compositions of the present invention can comprise, consist of, or consist essentially of the elements and limitations of the invention described herein, as well as any of the additional or optional ingredients, components, or limitations described herein. All percentages, parts and ratios are by weight of the total composition, unless otherwise specified. All such weights as they pertain to listed ingredients are based on the specific ingredient level and, therefore, do not include carriers or by-products that may be included in commercially available materials, unless otherwise specified.

I. Skin Treatment Agents The lotion compositions of the present invention comprise relatively low concentrations of a select combination of skin treatment agents that are capable of reducing and eliminating the occurrence of skin disorders that can result from contact between the skin and moisture-laden air, skin disorders resulting from prolonged moist human tissue that can occur from the skin being exposed to moisture or other body exudates, and/or skin disorders that are generated from contact between the skin and microbial or bacterial agents. The phrase "select combination of skin treatment agents" refers to the following combinations: a. hexamidine, zinc oxide, and niacinamide; b. hexamadine and zinc oxide; and c. hexamadine and niacinamide.

Surprisingly, the select combination of skin treatment agents can be included at low individual concentrations, relative to their use in the prior art, and still be effective. For example, the lotion compositions of the present invention can include hexamidine at a concentration of 0.1% or less by weight, zinc oxide at a concentration of 1% or less by weight, and niacinamide at a concentration of 2% or less by weight to achieve equal or superior benefits in the prevention and/or treatment of skin disorders as compared to known lotion compositions that generally comprise these skin treatment agents at higher levels. Similarly, the total effective concentration of the select combination of skin treatment agents in the compositions of the present invention are also relatively low. The total concentration of the select combination of skin treatment agents ranges from 0.002% to 10%, preferably from 0.01% to 5%, more preferably from 0.1% to 2% by weight of the lotion composition.

A. Hexamidine: The lotion compositions of the present invention comprise hexamidine skin treatment agent at concentrations ranging from 0.001% to 0.1%, from 0.005% to 0.1%, or even from 0.01% to 0.1% by weight of the composition. The hexamidine skin treatment agent suitable for use herein include those aromatic diamines which generally conform to the following formula:

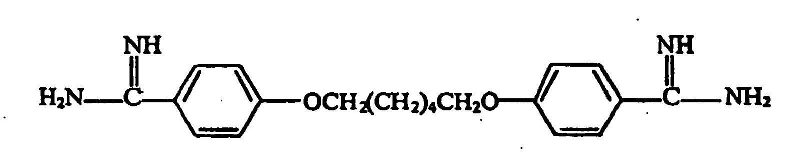

These aromatic diamines are referred to as 4,4'-[1,6-Hexanediylbis(oxy)]bisbenzenecarboximidamide; 4,4'-(hexamethylenedioxy)dibenzamidine; and 4,4'-diamidino-α,ω-diphenoxyhexane. The most popular employed form of hexamidine is the general category of hexmidine salts, which include acetate, salicylate, lactate, gluconate, tartarate, citrate, phosphate, borate, nitrate, sulfate, and hydrochloride salts of hexamidine. Specific nonlimiting examples of hexamidine salts include hexamidine isethionate, hexamidine diisethionate, hexamidine hydrochloride, hexamidine gluconate, and mixtures thereof. Hexamidine isethionate and hexamidine diisethionate are β-hydroxyethane sulfonate salts of hexamidine which are preferred for use herein as a skin treatment agent in the prevention and/or treatment of skin disorders. Hexamidine diisethionate is the most preferred hexamidine compound suitable for use as the skin treatment agent herein and is available from Laboratories Serolobilogiques (Pulnoy, France) and the Cognis Incorporation (Cincinnati, Ohio) under the tradename ELASTAB HP 100.

Hexamidine compounds are known as effective skin treatment agents that can control microbial growth that can lead to irritating and itching skin disorders. Therefore, these skin treatment agents are often referred to as antimicrobial agents. As used herein the term "antimicrobial agents" refer to materials which function to destroy or suppress the growth or metabolism of microbes, and include the general classification of antibacterial, antifungal, antiprotozoal, antiparasitic, and antiviral agents.

It has been found, however, that a low concentration (about 0.1% or less by weight) of hexamidine provides for improved reduction and/or prevention of skin irritating infections, especially when a low amount of hexamidine is combined with a low concentration of other antimicrobial agents such as zinc oxide and/or niacinamide. This combination of hexamidine and zinc oxide and/or niacinamide can be administered topically and internally at a total concentration less than an effective amount of an applied dosage of these individual compounds. As used herein the term "effective amount" refers to an amount with provides a therapeutic benefit with minimal or no adverse reaction in the reduction and/or prevention of any noticeable or unacceptable skin abnormality which causes irritating, acute, or chronic symptoms including itching and inflammation.

Other aromatic diamines are also suitable for use as a skin treatment agent herein. Such compounds include butamidine and derivatives thereof including butamidine isethionate; pentamidine and derivatives thereof including pentamidine isethionate and pentamidine hydrochloride; dibromopropamidine and derivatives thereof including dibromopropamidine isethionate; stilbamidine and derivatives thereof including hydroxystilbamidine, stilbamidine dihydrochloride, and stilbamidine isethionate; diaminodiamidines and derivatives thereof; and mixtures thereof.

B. Zinc Oxide: The lotion compositions of the present invention comprise zinc oxide skin treatment agent at concentrations ranging from 0.001% to 10%, preferably from 0.005% to 5%, more preferably from 0.005% to 2%, most preferably from 0.01% to 1% by weight of the composition. The zinc oxide skin treatment agent can be included in the compositions as an individual zinc oxide compound or a combination of zinc oxides, provided that the individual or combined zinc oxide can readily combine with the hexamidine and niacinamide skin treatment agents to provide antimicrobial benefits.

The zinc oxide skin treatment agent suitable for use herein include those inorganic white and yellowish-white powders that conform to the formula ZnO, and that are more fully described in The Merck Index, Eleventh Edition, entry 10050, p. 1599 (1989). Some particularly useful forms of zinc oxide include those that are manufactured and commercially available in average particle size diameters that range from about 1nm (nanometer) to 10µm (micrometer), alternatively from 10nm to 1µm or even from 20nm to 500nm. Surprisingly, the inventors have discovered that the use of the above mentioned, relatively small nanoparticle diameter size zinc oxide avoids undesirable skin or hair whitening that results from the transfer of the zinc oxide containing emollient from the topsheet of absorbent article to the wearer's body during product use. This is a particular benefit when the product is a panty liner, sanitary napkin, incontinence brief, or other absorbent article intended to be used by adults having hair in the region where the lotion composition will transfer.

Commercially available zinc oxides include the white zinc oxide powders sold under the tradename ULTRAFINE 350 which is commercially available from the Kobo Incorporation located in South Plainfield, New Jersey. Other suitable zinc oxide materials include a premix of zinc oxide and a dispersing agent such as polyhydroxystearic acid wherein this premix is available from the Uniqema Incorporation (Wilimington, Delaware) under the tradename Arlecel® P100; and a premix of zinc oxide and an isononyl isononanoate dispersing agent which is available from the Ikeda Incorporation (Island Park, New York) under the tradename Salacos® 99.

C. Niacinamide: The lotion compositions of the present invention comprise niacinamide skin treatment agent as an individual niacinamide or as a combination of niacinamides at a total niacinamide concentration ranging from 0.01% to 10%, preferably from 0.05% to 5%, more preferably from 0.2% to 2% by weight of the lotion composition. The niacinamide skin treatment agent provides for skin conditioning benefits as well as providing for increased efficacy of the skin treatment agents in controlling skin disorders.

Nonlimiting examples of niacinamide skin treatment agents suitable for use in the lotion compositions of the present invention include those niacinamide compounds that are amide derivatives of nicotinic acid, and that generally conform to the following formula:

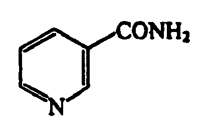

Niacinamide and nicotinic acid are also known as Vitamin B₃ and Vitamin B₅, whereas niacinamide is the commonly used active form. Niacinamide derivatives including salt derivatives are also suitable for use herein as a skin treatment agent. Nonlimiting specific examples of suitable niacinamide derivatives include nicotinuric acid and nicotinyl hydroxamic acid.

The niacinamide skin treatment agent can also be included in the composition as acidified niacinamide compounds. The process of acidifying niacinamide compounds is within the gambit of those skilled in the art, wherein one such technique involves dissolving niacinamide in an alcohol solution, adding while stirring an equal molar amount of a fatty acid such as stearic acid (e.g., mixing 1 part niacinamide to 2.4 parts stearic acid), and then air drying the mixture until the alcohol evaporates. A suitable stearic acid compound that can be used in the process of acidifying niacinamide is stearic acid sold under the tradename Emersol® 150 which is available from the Cognis Corporation.

Examples of the above niacinamide compounds are well known in the art and are commercially available from a number of sources, for example, the Sigma Chemical Company (St Louis, Missouri); ICN Biomedicals, Incorporation (Irvin, California); Aldrich Chemical Company (Milwaukee, Wisconsin); and Em Industries HHN (Hawthorne, New York).

D. Optional Components: Nonlimiting examples of optional suitable skin treatment actives useful in the present invention include allantoin; aluminum hydroxide gel; calamine; cysteine hydrochloride; racemic methionine; sodium bicarbonate; Vitamin C and derivatives thereof; protease inhibitors including serine proteases, metalloproteases, cysteine proteases, aspartyl proteases, peptidases, and phenylsulfonyl fluorides; lipases; esterases including diesterases; ureases; amylases; elastases; nucleases; guanidinobenzoic acid and its salts and derivatives; herbal extracts including chamomile; and mixtures thereof. Guanidinobenzoic acid and its salts and derivatives are more fully described in U.S. Patent 5,376,655, issued to Imaki et al. on December 27, 1994. These other suitable skin treatment actives are typically included at concentrations ranging from 0.001% to 10% by weight of the lotion composition.

Furthermore, one or more optional components known or otherwise effective for use in lotion compositions may be included provided that the optional components are physically and chemically compatible with the essential skin treatment and carrier components, or do not otherwise unduly impair product stability, aesthetics, or performance. Such optional components are typically included at concentrations ranging from 0.001% to 20% by weight of the compositions, and include materials such as water, skin conditioning agents, perfumes, deodorants, opacifiers, astringents, preservatives, emulsifying agents, film formers, stabilizers, proteins, lecithin, urea, colloidal oatmeal, pH control agents, and other Monographed materials that are deemed safe by the U.S. Food and Drug Administration (FDA) under 21 C.F.R. §347 for use on human skin. Other optional components for use in the lotion compositions of the present invention include fats or oils, or essential oils. These oils can be present at concentrations ranging from 0.0001% to 10% by weight of the compositions, and include materials such as Anise Oil, Balm Mint Oil" Bee Balm Oil, Birch Oil, Bitter Almond Oil, Bitter Orange Oil, Calendula Oil, California Nutmeg Oil, Caraway Oil, Chamomile Oil, Cinnamon Oil, Cloveleaf Oil, Clove Oil, Coriander Oil, Cypress Oil, Eucalyptus Oil, Fennel Oil, Gardenia Oil, Geranium Oil, Ginger Oil, Grapefruit Oil, Hyptis Oil, Juniper Oil, Kiwi Oil, Laurel Oil, Lavender Oil, Lemongrass Oil, Lemon Oil, Lovage Oil, Mandarin Orange Oil, Musk Rose Oil, Nutmeg Oil, Olibanurn, Orange Flower Oil, Orange Oil, Peppermint Oil, Pine Oil, Rose Hips Oil, Rosemary Oil, Rose Oil, Rue Oil, Sage Oil, Sandalwood Oil, Sassafras Oil, Spearmint Oil, Sweet Marjoram Oil, Sweet Violet Oil, Tea Tree Oil, Thyme Oil, Wild Mint Oil, Yarrow Oil, Ylang Ylang Oil, Apricot Kernel Oil, Avocado Oil, Babassu Oil, Borage Seed Oil, Butter, C12-Cl. Acid Triglyceride, Camellia Oil, Canola Oil, Caprylic/Capric/Lauric Triglyceride, Caprylic/Capric/Linoleic Triglyceride, Caprylic/Capric/Stearic Triglyceride, Caprylic/Capric305 Triglyceride, Carrot Oil, Cashew Nut Oil, Castor Oil, Cherry Pit Oil, Cocoa Butter, Coconut Oil, Cod Liver Oil, Corn Germ Oil, Corn Oil, Cottonseed Oil, C10-C1 Triglycerides, Evening Primrose Oil, Glyceryl Triacetyl Hydroxystearate, Glyceryl Triacetyl Ricinoleate, Glycosphingolipids, Grape Seed Oil, Hazelnut Oil, Human Placental Lipids, Hybrid Safflower Oil, Hybrid Sunflower Seed Oil, Hydrogenated Castor Oil, Hydrogenated Coconut Oil, Hydrogenated Cottonseed Oil, Hydrogenated C2-C1 Triglycerides, Hydrogenated Fish Oil, Hydrogenated Lard, Hydrogenated Menhaden Oil, Hydrogenated Mink Oil, Hydrogenated Orange Roughy Oil, Hydrogenated Palm Kernel Oil, Hydrogenated Palm Oil, Hydrogenated Peanut Oil, Hydrogenated Shark Liver Oil, Hydrogenated Soybean Oil, Hydrogenated Tallow, 315 Hydrogenated Vegetable Oil, Lard, Lauric/Palmitic/Oleic Triglyceride, Lanolin and Lanolin derivatives, Lesquerella Oil, Macadamia Nut Oil, Maleated Soybean Oil, Meadowfoam Seed Oil, Menhaden Oil, Mink Oil, Moringa Oil, Mortierella Oil, Oleic/Linoleic Triglyceride, Oleic/Paimitic/Lauric/Myristic/Linoleic Triglyceride, Oleostearine, Olive Husk Oil, Olive Oil, Oriental Lipids, Palm Kernel Oil, Palm Oil, 320 Peach Kernel Oil, Peanut Oil, Pentadesma Butter, Phospholipids, Pistachio Nut Oil, Rapeseed Oil, Rice Bran Oil, Safflower Oil, Sesame Oil, Shark Liver Oil, Shea Butter, Soybean Oil, Sphingolipids, Sunflower Seed Oil, Sweet Almond Oil, Tall Oil, Tallow, Tribehenin, Tricaprin, Tricaprylin, Triheptanoin, C10 Fatty Acids: Arachidic Acid, Behenic Acid, Capric Acid, Caproic Acid, 330 Caprylic Acid, Coconut Acid, Corn Acid, Cottonseed Acid, Hydrogenated Coconut Acid, Hydrogenated Menhaden Acid, Hydrogenated Tallow Acid, Hydroxystearic Acid, Isostearic Acid, Lauric Acid, Linoleic Acid, Linolenic Acid, Myristic Acid, Oleic Acid, Palmitic Acid, Palm Kernel Acid, Pelargonic Acid, Ricinoleic Acid, Soy Acid, Stearic Acid, Tallow Acid, Undecanoic Acid, Undecylenic Acid, Wheat Germ Acid, and the like, as well as mixtures thereof. Specific optional lotion conditioning agents found useful in the present invention include panthenol, glycerine, and chamomile oil which are described in detail hereinbelow. Panthenol: Where included, panthenol typically comprises from 0.001% to 10%, preferably from 0.005% to 5%, more preferably from 0.05% to 1% by weight of the lotion composition. The optional panthenol skin conditioning agent provides for skin emolliency benefits that can leave the skin feeling smooth, soothing, and soft during and after interaction of the skin tissues with the skin treatment agents. The lotion compositions of the present invention can include an individual panthenol compound or a mixture of panthenol compounds.

Nonlimiting examples of panthenol include those panthenol compounds which are alcohol or ester derivatives of pantothenic acid. Pantothenic acid is a member of the B complex family and is often referred to as Vitamin B₃. Like pantothenic acid, the panthenol alcohol derivatives of this acid can exist as stereoisomers, for example, the D(+) form, the L(-) form, the racemate, and mixtures of the D(+) and L(-) forms. Specific examples of panthenol include, but are not limited to, D-panthenol (a.k.a. dexpanthenol), and dl-panthenol. Panthenol is more fully described in The Merck Index, Eleventh Edition, entry 2924, p. 464 (1989). Examples of commercially available panthenol include D-panthenol which is available from Roche Vitamins Incorporation (Nutley, New Jersey), a subsidiary of F. Hoffman LaRoche, Ltd.
Glycerine: Where included, the lotion compositions comprise the preferred optional glycerine skin conditioning agent at concentrations ranging from 0.01% to 10%, preferably from 0.02% to 5%, more preferably from 0.05% to 2% by weight of the lotion composition. The optional glycerine skin conditioning agent also provides for skin emolliency benefits such as smooth, soothing, and soft feeling skin, as well as being a dispersing agent for the niacinamide skin treatment agent.

Glycerine is a C3 monohydric alcohol that is also referred to as glycerol and 1,2,3-propanetriol. Glycerine derivatives are also suitable for use as an optional skin conditioning agent herein wherein such derivatives include polyglycerols having from about 2 to about 16 repeating glycerol moieties. A specific example of a suitable glycerine skin conditioning agent is Glycerine, USP Kosher® which is commercially available from the Procter & Gamble Company located in Cincinnati, Ohio. Chamomile: The lotion compositions comprise the preferred optional chamomile oil at concentrations ranging from 0.0001% to 10%, preferably from 0.001% to 5%, more preferably from 0.005% to 2% by weight of the lotion composition. The optional chamomile oil skin conditioning agent also provides for skin benefits such as soothing. Chamomile oil is commonly prepared as an oil extract of chamomile flowers. An example of a commercially available chamomile oil include Phytoconcentrol Chamomile which is available from Dragoco Incorporation (Totowa, New Jersey).

II. Carrier: The lotion compositions of the present invention comprise a carrier for the skin treatment agents. The carrier can be included in the compositions as an individual carrier or a combination of carrier ingredients, provided that the total carrier concentration is sufficient to provide transfer and/or migration of the skin treatment agents onto the skin. The carrier can be a liquid, solid, or semisolid carrier material, or a combination of these materials, provided that the resultant carrier forms a homogenous mixture or solution at selected processing temperatures for the resultant carrier system and at processing temperatures for combining the carrier with the skin treatment agents in formulating the lotion compositions herein. Processing temperatures for the carrier system typically range from 60°C to 90°C, more typically from 70°C to 85°C, even more typically from 70°C to 180°C.

The lotion compositions of the present invention typically comprise the carrier at a total carrier concentration ranging from 60% to 99.9%, preferably from 70% to 98%, more preferably from 80% to 97% by weight of the lotion composition. Suitable carrier compounds include petroleum-based hydrocarbons having from 4 to 32 carbon atoms, fatty alcohols having from 12 to 24 carbon atoms, polysiloxane compounds, fatty acid esters, alkyl ethoxylates, lower alcohols having from 1 to 6 carbon atoms, low molecular weight glycols and polyols, fatty alcohol ethers having from 12 to 28 carbon atoms in their fatty chain, lanolin and its derivatives, glyceride and its derivatives including acetoglycerides and ethoxylated glycerides of C12-C28 fatty acids, and mixtures thereof. In combination with, the carrier may also be composed of polysiloxane compounds non-limiting examples include dimethicones (1-100,000,000 centistoke), cyclomethicones, alkylated silicones (hair conditioning agents), silicone gums, silicone gels, silicone waxes, copolymers of silicone (vinyl dimethicone polymers, phenyl vinyl dimethicone polymers, alkylated silicone polymers, polyethylene oxide / silicone copolymers, polyethylene oxide / alkyl silicone copolymers), and mixtures thereof.

Nonlimiting examples of suitable petroleum-based hydrocarbons having from 4 to 32 carbon atoms include mineral oil, petrolatum, isoparaffins, various other branched chained hydrocarbons, and combinations thereof. Mineral oil is also known as "liquid petrolatum", and usually refers to less viscous mixtures of hydrocarbons having from 16 to 20 carbon atoms. Petrolatum is also known as "mineral wax", "petroleum jelly", and "mineral jelly", and usually refers to more viscous mixtures of hydrocarbons having from 16 to 32 carbon atoms. An example of commercially available petrolatum include petrolatum sold as Protopet® 1S which is available from the Witco Corporation located in Greenwich, Connecticut.

Nonlimiting examples of suitable fatty alcohols having from 12 to 24 carbon atoms include saturated, unsubstituted, monohydric alcohols or combinations thereof, which have a melting point less than 110°C, preferably from 45°C to 110°C. Specific examples of fatty alcohol carriers for use in the lotion compositions of the present invention include, but are not limited to, cetyl alcohol, stearyl alcohol, cetearyl alcohol, behenyl alcohol, arachidyl alcohol, lignocaryl alcohol, and combinations thereof. Examples of commercially available cetearyl alcohol is Stenol 1822 and behenyl alcohol is Lanette 22, both of which are available from the Cognis Corporation located in Cincinnati, Ohio.

Nonlimiting examples of suitable fatty acid esters include those fatty acid esters derived from a mixture of C₁₂-C₂₈ fatty acids and short chain (C₁-C₈, preferably C₁-C₃) monohydric alcohols preferably from a mixture of C₁₆-C₂₄ saturated fatty acids and short chain (C₁-C₈, preferably C₁-C₃) monohydric alcohols. Representative examples of such esters include methyl palmitate, methyl stearate, isopropyl laurate, isopropyl myristate, isopropyl palmitate, ethylhexyl palmitate, and mixtures thereof. Suitable fatty acid esters can also be derived from esters of longer chain fatty alcohols (C₁₂-C₂₈, preferably C₁₂-C₁₆) and shorter chain fatty acids such as lactic acid, specific examples of which include lauryl lactate and cetyl lactate.

Nonlimiting examples of suitable alkyl ethoxylates include C₁₂-C₂₂ fatty alcohol ethoxylates having an average degree of ethoxylation of from 2 to 30. Nonlimiting examples of suitable lower alcohols having from about 1 to about 6 carbon atoms include ethanol, isopropanol, butanediol, 1,2,4-butanetriol, 1,2 hexanediol, ether propanol, and mixtures thereof. Nonlimiting examples of suitable low molecular weight glycols and polyols include ethylene glycol, polyethylene glycol (e.g., Molecular Weight 200-600 g/mole), butylene glycol, propylene glycol, polypropylene glycol (e.g., Molecular Weight 425-2025 g/mole), and mixtures thereof. A more detailed description of carrier ingredients including suitable hydrocarbons, polysiloxane compounds, and fatty alcohol ethoxylates can be found in U.S. Patent No. 5,643,588, issued July 1, 1997 to Roe et al. entitled "Diaper Having A Lotioned Topsheet".

In one embodiment, the carrier comprises a combination of one or more petroleum-based hydrocarbons and one or more fatty alcohols described hereinabove. When one or more petroleum-based hydrocarbons having from about 4 to about 32 carbon atoms are used in combination with one or more fatty alcohols having from 12 to 22 carbon atoms, the petroleum-based hydrocarbons are included at total concentrations ranging from 20% to 99%, preferably from 30% to 85%, more preferably from 40% to 80% by weight of the lotion composition; wherein the fatty alcohols are included at total concentrations ranging from 0.2% to 65%, preferably from 1% to 50%, more preferably from 2% to 40% by weight of the lotion composition.

It is believed that a petroleum-based carrier system comprising C₄-C₃₂ hydrocarbons, C₁₂-C₂₂ fatty alcohols, and fumed silica provides a homogeneous mixture of the carrier, skin treatment agents, and any optional ingredients wherein this homogeneous mixture ensures sufficient contact between the skin and skin treatment agents to result in effective prevention and treatment of skin disorders. The fumed silica suitable for inclusion in the preferred petroleum-based carrier system, or with any other carrier described herein, includes colloidal pyrogenic silica pigments which are sold under the Cab-O-Sil® tradename, and which are commercially available from the Cabot Corporation located in Tuscola, Illinois. These colloidal pyrogenic silica pigments are submicroscopic particulated pyrogenic silica pigments having mean particle sizes ranging from 0.1 microns to 100 microns. Specific examples of commercially available Cab-O-Sil® silica pigments include Cab-O-Sil® TS-720 (a polydimethylsiloxane treated fumed silica), Cab-O-Sil® TS-530 (a trimethyl silanized fumed silica), and Cab-O-Sil® TS-610 (a dimethyldisilanized fumed silica). The fumed silica provides the lotion compositions with desired viscosity or thickening properties, and is typically included at concentrations ranging from 0.01% to 15%, preferably from 0.1% to 10%, more preferably from 1% to 5% by weight of the lotion composition.

The fumed silica can be used alone or in combination with other optional viscosity or thickening agents such as talc, bentonites including treated bentonites, hectorites including treated hectorites, calcium silicates including treated calcium silicates, magnesium silicates, magnesium aluminum silicates, zinc stearates, sorbitol, colloidal silicone dioxides, spermaceti, carnuba wax, beeswax, candelilla wax, paraffin wax, microcrystalline wax, castrol wax, ceresin, esparto, ouricuri, rezowax, polyethylene wax, C₁₂-C₂₄ fatty acids, polyhydroxy fatty acid esters, polyhydroxy fatty acid amides, polymethacrylate polymers, polymethacrylate and styrene copolymers, and combinations thereof. These other optional viscosity modifying or thickening agents are also included at total concentrations ranging from 0.01% to 15% by weight of the lotion composition. A nonlimiting specific example of another suitable viscosity or thickening agent include bentonite sold as Bentone® 38 which is available from the Rheox Incorporation.

The carrier must be hydrophobic. Further, it is preferable that the lotion composition of the present invention comprise no surfactant. Therefore, the lotion has a level of hydrophobicity at least as great as that of the topsheet, and the hydrophobicity of the lotion is primarily due to the lack of a surfactant component. In order to raise the wettability of the hydrophobic carrier one may add a wetting agent such as polyoxyethylene alkyl ethers, alkyl ethoxylates, alkylethoxylated amines, polyethylene glycol esters, and/or sorbitan fatty acid esters generally having a low degree of ethoxylation and HLB values below 7. Suitable additives will be miscible with the carrier so as to form a homogenous mixture. Because of possible skin sensitivity of those using the catamenial device of the present invention, these wetting agents should also be relatively mild and non-irritating to the skin. Typically, these wetting agents are nonionic to be not only non-irritating to the skin, but also to avoid other undesirable effects on any underlying tissue laminate structure, e.g., reductions in tensile strength. Suitable wetting agents will have HLB values below 7.

Non-limiting specific examples of a suitable wetting agents includes nonyl phenol or or polyoxyethylene nonyl phenyl ether (20 of ethoxylation; HLB of 5.7), octyl phenol or polyoxyethylene octyl phenyl ether (10 of ethoxylation; HLB of 3.5), stearyl alcohol or polyoxyethylene stearyl ether (20 of ethoxylation; HLB of 4.9), stearyl amine or polyoxyethylene stearyl amine (20 of ethoxylation; HLB of 4.9), polyethylene glycol 200 dilaurate (HLB 5.9), polyethylene glycol 200 distearate (HLB 4.8), sorbitan monostearate ('Span 60' having HLB 4.7), sorbitan tristearate ('Span 65' having HLB 2.1), sorbitan monooleate ('Span 80' having HLB 4.3), sorbitan trioleate ('Span 85' having HLB 1.8), each of which are available form Cell Chemical Company (Inchon, Korea) or Uniqema (New Castle, Delaware, USA).

The amount of wetting agent required to increase the wettability of the lotion composition to a desired level will depend upon its HLB value and HLB level of the carrier used, and like factors. The lotion composition can comprise from 1 to 25% of the wetting agent when needed to increase the wettability properties of the composition.

III. Absorbent Article The lotion compositions of the present invention are preferably transferred to the skin from application of the compositions onto a catamenial device. These products may comprise a topsheet, a backsheet, and an absorbent core positioned between the topsheet and backsheet; each component having a body-or wearer-contacting surface and a garment surface. The terms "body-contacting surface" and "wearer-contacting surface" are used interchangeably herein and refer to one or more surfaces of any article component that is intended to be worn or positioned toward or adjacent the body of the wearer/user for contact between the wearer/user and the article's surface at some time during the use period. The term "garment surface" as used herein refers to the outer or exterior surface of any article component that is intended to be worn or positioned adjacent a wearer's undergarments, or in the case of an absorbent article which is not worn by the user, the garment surface is typically positioned adjacent a user's hand or other implement assisting in the use of the absorbent article. As used herein, the term "wearer" and "user" are used interchangeably as the present invention contemplates absorbent articles which may not be intended to be worn, but rather used to absorb bodily exudates while transferring the lotion compositions of the present invention.

A. Topsheet: The absorbent article may comprise any known or otherwise effective topsheet, such as one which is compliant, soft feeling, and non-irritating to the wearer's skin. Suitable topsheet materials include a liquid pervious material that is oriented towards and contacts the body of the wearer permitting bodily discharges to rapidly penetrate through it without allowing fluid to flow back through the topsheet to the skin of the wearer. The topsheet, while being capable of allowing rapid transfer of fluid through it, also provides for the transfer or migration of the lotion composition onto an external or internal portion of a wearer's skin. A suitable topsheet can be made of various materials such as woven and nonwoven materials; apertured film materials including apertured formed thermoplastic films, apertured plastic films, and fiber-entangled apertured films; hydro-formed thermoplastic films; porous foams; reticulated foams; reticulated thermoplastic films; thermoplastic scrims; or combinations thereof.

Apertured film materials suitable for use as the topsheet include those apertured plastic films that are non-absorbent and pervious to body exudates and provide for minimal or no flow back of fluids through the topsheet. Nonlimiting examples of other suitable formed films, including apertured and non-apertured formed films, are more fully described in U.S. Patent No. 3,929,135, issued to Thompson on December 30, 1975; U.S. Patent No. 4,324,246, issued to Mullane et al. on April 13, 1982; U.S. Patent No. 4,324,314, issued to Radel et al. on August 3, 1982; U.S. Patent No. 4,463,045, issued to Ahr et al. on July 31, 1984; U.S. Patent No. 5,006,394, issued to Baird on April 9, 1991; U.S. Patent No. 4,609,518, issued to Curro et al. on September 2, 1986; and U.S. Patent No. 4,629,643, issued to Curro et al. on December 16, 1986. Commercially available formed filmed topsheets include those topsheet materials marketed by the. Procter&Gamble Company (Cincinnati, Ohio) under the DRI-WEAVE® tradename.

Nonlimiting examples of woven and nonwoven materials suitable for use as the topsheet include fibrous materials made from natural fibers, modified natural fibers, synthetic fibers, or combinations thereof. These fibrous materials can be either hydrophilic or hydrophobic, but it is preferable that the topsheet be hydrophobic or rendered hydrophobic. As an option portions of the topsheet can be rendered hydrophilic, by the use of any known method for making topsheets containing hydrophilic components. One such method include treating an apertured film component of a nonwoven/apertured thermoplastic formed film topsheet with a surfactant as described in U.S. Patent No. 4,950,264, issued to Osborn on August 21, 1990. Other suitable methods describing a process for treating the topsheet with a surfactant are disclosed in U.S. Patent Nos. 4,988,344 and 4,988,345, both issued to Reising et al. on January 29, 1991. The topsheet can comprise hydrophilic fibers, hydrophobic fibers, or combinations thereof.

When the topsheet comprises a nonwoven fibrous material in the form of a nonwoven web, the nonwoven web may be produced by any known procedure for making nonwoven webs, nonlimiting examples of which include spunbonding, carding, wet-laid, air-laid, meltblown, needle-punching, mechanical entangling, thermomechanical entangling, and hydroentangling. A specific example of a suitable meltblown process is disclosed in U.S. Patent No. 3,978,185, to Buntin et al., issued August 31, 1976.

Other suitable nonwoven materials include low basis weight nonwovens, that is, nonwovens having a basis weight of from 18 g/m² to 25 g/m². An example of such a nonwoven material is commercially available under the tradename P-8 from Veratec, Incorporation, a division of the International Paper Company located in Walpole, Massachusetts.

B. Backsheet: The catamenial device of the present invention also comprises a backsheet. The backsheet can be any known or otherwise effective backsheet material, provided that the backsheet prevents external leakage of exudates absorbed and contained in the catamenial device. Flexible materials suitable for use as the backsheet include, but are not limited to, woven and nonwoven materials, laminated tissue, polymeric films such as thermoplastic films of polyethylene and/or polypropylene, composite materials such as a film-coated nonwoven material, or combinations thereof.

C. Absorbent Core: The catamenial device also comprises an absorbent. The absorbent core is typically positioned between the topsheet and the backsheet. As used herein, the term "absorbent core" refers to a material or combination of materials suitable for absorbing, distributing, and storing aqueous fluids such as urine, blood, menses, and water found in body exudates. The size and shape of the absorbent core can be altered to meet absorbent capacity requirements, and to provide comfort to the wearer/user. The absorbent core suitable for use in the present invention can be any liquid-absorbent material known in the art for use in absorbent articles, provided that the liquid-absorbent material can be configured or constructed to meet absorbent capacity requirements. Nonlimiting examples of liquid-absorbent materials suitable for use as the absorbent core include comminuted wood pulp which is generally referred to as airfelt; creped cellulose wadding; absorbent gelling materials including superabsorbent polymers such as hydrogel-forming polymeric gelling agents; chemically stiffened, modified, or crosslinked cellulose fibers; meltblown polymers including coform; synthetic fibers including crimped polyester fibers; tissue including tissue wraps and tissue laminates; capillary channel fibers; absorbent foams; absorbent sponges; synthetic staple fibers; peat moss; or any equivalent material; or combinations thereof.

IV. Methods of Treating the Skin: The present invention also relates to methods of treating the skin with the lotion compositions described herein. Generally, a safe and effective amount of the lotion composition is applied to an absorbent article described herein wherein such safe and effective amounts include applying from 0.0015 mg/cm² (0.01 mg/in²) to 100.5 mg/cm² (100 mg/in²), preferably from 0.003 mg/cm² (0.02 mg/in²) to 12.4 mg/cm² (80 mg/in²), more preferably from 0.02 mg/cm² (0.015 mg/in²) to 7.75 mg/cm² (50 mg/in²), of the lotion composition to the absorbent article.

Typically, a safe and effective amount of the lotion compositions of the present invention is applied to an absorbent article such that at least 0.00015 mg/cm² (0.001 mg/in²) to 15.5 mg/cm² (100 mg/in²), preferably from 0.0006 mg/cm² (0.004 mg/in²) to 11 mg/cm² (72 mg/in²), more preferably from 0.005 mg/cm² (0.03 mg/in²) to 6.2 mg/cm² (40 mg/in²), of the composition is transferred to the skin during a single use of an absorbent article which is typically about a three hour period. Absorbent articles are generally changed every three to six hours during the day and once for overnight protection, resulting in at least a safe and effective amount of from 0.00045 mg/cm² (0.003 mg/in²) to 124 mg/cm² (800 mg/in²), preferably from 0.0018 mg/cm² (0.012 mg/in²) to 88 mg/cm² (576 mg/in²), more preferably from 0.015 mg/cm² (0.09 mg/in²) to 49.6 mg/cm² (320 mg/in²), of the lotion composition being administered within a one day interval (24 hour period). However, the transfer of the lotion compositions of the present invention onto a wearer's skin via an absorbent article described herein can occur for one day, several days, weeks, months, or years at appropriate intervals provided that safe and effective amounts of the lotion compositions are administered to deliver the skin treatment benefits described herein.

The lotion compositions of the present invention can be applied to the absorbent articles by any known or otherwise effective technique for distributing a lotion composition onto an absorbent product such as a disposable absorbent article. Nonlimiting examples of methods of applying the lotion compositions onto an absorbent article include spraying, printing (e.g., flexographic printing), coating (e.g., contact slot coating and gravure coating), extrusion, or combinations of these application techniques. The application of the lotion compositions onto an absorbent article facilitates the transfer or migration of the lotion compositions onto the skin for administration and/or deposition of the lotion compositions, resulting in a safe and effective amount of the compositions being applied for improved prevention and reduction of skin disorders. Therefore, the safe and effective amount of the lotion composition that will transfer or migrate to the skin will depend on factors such as the type of lotion composition that is applied, the portion of the body contacting surface where the lotion composition is applied, and the type of absorbent article used to administer the lotion composition.

Any suitable method can be used in determining the amount of a lotion composition described herein that is transferred to the skin of a wearer during use of an absorbent article containing the composition. An example of specific methods for the calculation of transfer amounts of lotion compositions include Gas Chromatographic and other quantitative analytical procedures that involve the analysis of in vivo skin analog materials. A suitable Gas Chromatographic procedure is more fully described in WO 99/45973, Donald C. Roe et al, published September 16, 1999..

V. Method of Manufacture: The lotion compositions of the present invention may be prepared by any known or otherwise effective technique, suitable for providing a lotion composition comprising the essential skin treatment agents defined herein. In general, the lotion compositions are prepared by first making a carrier system comprising suitable carriers such as petrolatum and behenyl alcohol in combination with a fumed silica thickening agent. Next, a mixture comprising the skin treatment agents and any optional ingredients such as optional skin conditioning agents are added to the carrier system at a melt mix temperature of 80°C. Although the carrier system, skin treatment agents, and any optional ingredients are typically processed at a temperature of 80°C, these materials can be processed at temperatures ranging from 60°C to 90°C, preferably from 70°C to 90°C. The resultant lotion composition is subsequently applied to a topsheet component of an absorbent article using a contact applicator such as a Nordsen EP 11-12-02.

The lotion compositions of the present invention are prepared such that the compositions can be applied to an absorbent article to result in safe and effective amounts of the compositions being transferred onto the skin of a wearer of the absorbent article. Therefore, the lotion compositions preferably have a product consistency such that they are relatively immobile and localized on the wearer-contacting surface of the absorbent article at ambient conditions, are readily transferable to the wearer at body temperature, and yet are not completely liquid under extreme storage conditions. In other words, the lotion compositions are solids or semisolids at ambient conditions (about 25°C) and/or body temperature (about 37°C) so that the compositions are easily transferred onto the skin by way of normal contact, wearer motion, and/or body heat. The consistency of the lotion compositions can be measured according to ASTM D5 test method which involves the use of a penetrometer to measure consistency. Typically, the lotion compositions of the present invention have a consistency of from 10 to 300, preferably from 20 to 250, more preferably from 30 to 200, as measured at 40°C according to the test procedure outlined in ASTM D5 test method.

The solid or semisolid consistency of the lotion compositions provide for relatively low levels of the compositions to be applied to the absorbent articles to impart the desired lotion benefits. By "semisolid" is meant that the compositions have a rheology typical of pseudoplastic or plastic liquids such that the compositions remain relatively stationary in a desired location on the absorbent article, and do not have a tendency to flow or migrate to undesired locations of the article. The solid lotion compositions of the present invention likewise can remain in a particular location and not flow or migrate to undesired locations of the article. These solid and semisolid lotion compositions have viscosities high enough to keep the compositions localized on an intended location of the article, but not so high as to impede transfer to the wearer's skin. Typically, final products of solid and semisolid lotion compositions have viscosities ranging from 1000 Pa·s (1.0 x 10⁶ centipoise) to 1 x 10⁷ Pa·s (1.0 x 10¹⁰) centipoise under shear stress conditions of 300 Pa (3 x 10³ dynes/cm²) at 40°C (the shear stress applied to the compositions while the absorbent article is in storage or transported at temperature conditions of 40°C).

However, the solid and semisolid lotion compositions can be made flowable for transfer or migration of the compositions onto the skin by applying shear stress that results in deformation of the compositions. The shear stress applied at least once during wear of the absorbent article under temperature conditions of 40°C is typically at 100000 Pa·s (1.0 x10⁶ dynes/cm²), and this shear stress can result in the lotion compositions having a viscosity of from 0,01 Pa·s (1.0x10¹ centipoise) to 100 Pa·s (1.0x 10⁵ centipoise). It is believed that the lotion compositions achieve the lower viscosity values under applied shear stress due to the fact that, while the compositions contain solid components, they also contain liquid materials. During wear of an absorbent article described herein, it is desirable to achieve a low viscosity for obtaining sufficient lubrication between the wearer's skin and the body contacting surface of the article to result in effective transfer of the lotion composition onto the wearer's skin. Viscosity at various shear stress can be measured using rheometers known in the art such as the Rheometer SR-2000 available from Rheometrics Incorporation.

The lotion compositions are typically applied to the topsheet of an absorbent article for delivery of the lotion composition onto an external or internal surface of the skin. The lotion composition can be applied to other areas of the absorbent article wherein these areas include wings, side panels, the absorbent core, any secondary layer intermediate the core and topsheet, or any other region of the absorbent article.

Processes for assembling absorbent articles such as the disposable absorbent articles described herein include conventional techniques known in the art for constructing and configuring disposable absorbent articles. For example, the backsheet and/or the topsheet can be joined to the absorbent core or to each other by a uniform continuous layer of adhesive, a patterned layer of adhesive, or an array of separate lines, spirals, or spots of adhesive. Adhesives which have been found to be satisfactory are manufactured by H. B. Fuller Company of St. Paul, Minnesota under the designation HL-1258 or H-2031.

The lotion compositions of the present invention can also be delivered onto the skin by incorporating the compositions into aerosol dispensers, trigger spray dispensers, pump spray dispensers, jars, stick dispensers, cotton balls, patches, sponges, and any other type of known or otherwise effective delivery vehicle.

### EXAMPLES

The examples are given solely for the purpose of illustration. All exemplified concentrations are weight-weight percents, unless otherwise specified.
Example_I: The compositions exemplified hereinbelow in Table 1 are representative of carrier systems of the lotion compositions of the present invention. The carrier systems are generally prepared by combining, by weight, petrolatum and a fatty alcohol such as behenyl alcohol, and then heating the mixture while stirring to a temperature of about 80°C using a low speed propeller mixer. Next, viscosity or thickening agents are added to the mixture to shear mix the ingredients into a final carrier system. Suitable viscosity or thickening agents include beheneth-10, fumed silica, bentonite, and steareth-2, wherein the viscosity or thickening agents are used alone or in combination. The ingredients can be shear mixed at 11,000 revolutions per minute (rpm) using an IKA Ultra Turrax Shear Mixer.

Alternatively, the petrolatum, fatty alcohol, and viscosity or thickening agent can be combined, heated with stirring at 80°C to melt the ingredients, and then mixed into a final carrier system using a high speed blade mixer such as the Tokusyu Kika TK Robo Mics which operates at 5,000 rpm.

**Table 1: Carrier Systems**

| Component | Sample 1 | Sample 2 | Sample 3 | Sample 4 | Sample 5 |
|---|---|---|---|---|---|
| | (Wt. %) | (Wt. %) | (Wt. %) | (Wt. %) | (Wt. %) |
| Petrolatum¹ | 85.4 | 78.1 | 70.0 | 70.0 | 80 |
| Behenyl Alcohol² | 11.0 | 8.7 | -- | 20.0 | 10 |
| Cetearyl Alcohol³ | | | 30.0 | -- | |
| Beheneth-10⁴ | -- | 10.0 | -- | -- | |
| Fumed Silica⁵ | 3.6 | 3.2 | -- | -- | 3.5 |
| Bentonite⁶ | -- | -- | -- | 10.0 | |
| Span 60⁷ | | | | | 6.5 |

| | | | | | |
|---|---|---|---|---|---|
| Wt. % - weight percent 1 - petrolatum available as Protopet® IS from the Witco Corporation 2 - behenyl alcohol available as Lanette® 22 from the Cognis Corporation 3 - cetearyl alcohol available as Stenol® 1822 from the Cognis Corporation 4 - bebeneth-10 available as Mergital® B10 from the Cognis Corporation 5 - fumed silica available as Cabosil® TS-720 from the Cabot Corporation 6 - bentonite available as Bentone® 38 from the Rheox Incorporation 7 - steareth-2 available as Brij® 762 from the Uniqema Corporation | | | | | |

Examples II-IX: The following Examples II-IX illustrated hereinbelow in Table 2 are representative of lotion compositions of the present invention that include the carrier systems identified in Table 1. The lotion compositions are prepared by formulating a premix solution of the zinc oxide skin treatment agent and adding the zinc oxide premix to the other skin treatment agents and any optional ingredients such as panthenol and glycerin, or by formulating a skin treatment solution of hexamidine and niacinamide skin treatment agents and any optional ingredients. The skin treatment solution is then added to a carrier system such as those described in Table 1, wherein the skin treatment solution and carrier system is heated while stirring to a temperature of about 80°C. All ingredients are included by weight of the lotion compositions. These lotion compositions are especially effective in the control of skin disorders such as skin erythema, malodor, and skin bacterial infections.

**Table 2: Lotion Compositions**

| Component | Ex. II | Ex. III | Ex. IV | Ex. V | Ex. VI | Ex. VII | Ex. | Ex IX |
|---|---|---|---|---|---|---|---|---|
| | (Wt.%) | (Wt. | (Wt. | (Wt. | (Wt. | (Wt. | VIII | (Wt. |
| | | %) | %) | %) | %) | %) | (Wt. | %) |
| | | | | | | | %) | |
| Sample 1 | 97.1 | 98.1 | 89.8 | -- | -- | -- | -- | -- |
| Sample 2 | -- | -- | -- | 96.2 | 99.7 | -- | -- | -- |
| Sample 3 | -- | -- | -- | -- | -- | 95.7 | -- | -- |
| Sample 4 | -- | -- | -- | -- | -- | -- | 97.3 | -- |
| Sample 5 | -- | -- | -- | -- | -- | -- | -- | 97.8 |
| ZnO Premix⁸ | 0.7 | 0.2 | 7.1 | 0.75 | 0.2 | -- | -- | -- |
| Hexamidine ⁹ | 0.1 | 0.1 | 0.1 | 0.05 | 0.1 | 0.1 | 0.05 | 0.1 |
| Panthenol¹⁰ | 0.5 | 0.5 | 0.5 | 0.5 | -- | 0.5 | 0.25 | -- |
| Glycerine¹¹ | 0.1 | 0.1 | -- | -- | -- | -- | -- | 0.1 |
| Niacinamide¹² | 1.0 | 1.0 | 2.0 | 2.0 | -- | -- | -- | 2.0 |
| Acidified Niacinamide¹³ | -- | -- | -- | -- | -- | 3.7 | 1.9 | -- |
| Chamomile ¹⁴ | 0.5 | -- | 0.5 | 0.5 | -- | -- | 0.5 | -- |

| | | | | | | | | |
|---|---|---|---|---|---|---|---|---|
| 8 - Zinc oxide premix comprising 70% zinc oxide mixture of UL TRAFINE® 350 zinc oxide available from the Kobo Incorporation, Arlecel® P100 available from the Uniqema Incorporation, and Salacos® 99 available from the Ikeda Incorporation 9 - hexamidine available as hexamidine diisethionate from Laboratories Serolobilogiques under the tradename ELASTAB® HP100 10 - panthenol available as D-panthenol from Roche Vitamins Incorporation 11 - glycerine available as Glycerine, USP Kosher® from the Procter & Gamble Company 12 - niacinamide available from Em Industries HHN 13 - acidified niacinamide made by reacting niacinamide with stearic acid 14 - chamomile available as Phytoconcentrol Chamomile from Dragoco | | | | | | | | |

The lotion composition of Example II is subsequently applied to the entire wearer-contacting surface of a DRI-WEAVE topsheet of a sanitary pad product such as Allways Wing Regular Long manufactured by the Procter & Gamble Company. To deliver a safe and effective amount of the lotion composition onto the skin, about 0.4 mg/cm² (2.6 mg/in²) of the lotion composition is applied to the topsheet using a Meltex EP45 hot melt applicator having a head operating temperature of about 90°C.

The lotion composition of Example III is subsequently applied by spraying the composition onto the entire wearer-contacting surface of a DRI-WEAVE topsheet of a sanitary pad product such as Envive Miniform manufactured by the Procter & Gamble Company. To deliver a safe and effective amount of the lotion composition onto the skin, about 4.0 mg/cm² (25.8 mg/in²) of the lotion composition is applied to the topsheet using a hot melt pneumatic Dynatec E84B1758 spray head having a head operating temperature of about 90°C and an atomization pressure of about 16 kiloPascals (kPa).

The lotion composition of Example IV is subsequently applied by slot coating (Nordsen EP 11-12-02) striped configurations of the composition onto the wearer-contacting surface of a hydrophobic spunbond bicomponent polyethylene / polypropylene topsheet (BBA, Washougal, WA) of a sanitary pad product. To deliver a safe and effective amount of the lotion composition onto the skin, the lotion composition is applied to the topsheet in a striped configuration wherein the striped configuration comprises at least two stripes each being 40 millimeters (mm) wide x 200 mm long and having about 0.8 mg/cm² (5.2 mg/in²) of the composition applied thereon.

The lotion composition of Example V is subsequently applied by spraying striped configurations of the composition onto the wearer-contacting surface of a DRI-WEAVE topsheet of a panty liner product such as Alldays Regular manufactured by the Procter & Gamble Company. To deliver a safe and effective amount of the lotion composition onto the skin, the lotion composition is applied to the topsheet in a striped configuration wherein the striped configuration comprises at least two stripes each being 40 millimeters (mm) wide x 200 mm long and having about 0.6 mg/cm² (3.9 mg/in²) of the composition applied thereon. The lotion composition is applied to the topsheet using a hot melt pneumatic Dynatec E84B 1758 spray head having a head operating temperature of about 90°C and an atomization pressure of about 16 kiloPascals (kPa).

The lotion composition of Example VI is subsequently applied to the entire wearer-contacting surface of a DRI-WEAVE topsheet of a panty liner product such as Alldays Regular manufactured by the Procter & Gamble Company. To deliver a safe and effective amount of the lotion composition onto the skin, about 0.2 mg/cm² (1.3 mg/in²) of the lotion composition is applied to the topsheet using a Meltex EP45 hot melt applicator having a head operating temperature of about 90°C.

The lotion composition of Example VII is subsequently applied by spraying the composition onto the entire wearer-contacting surface of a DRI-WEAVE topsheet of sanitary pad product such as Envive Miniform manufactured by the Procter & Gamble Company. To deliver a safe and effective amount of the lotion composition onto the skin, about 1.0 mg/cm² (6.5 mg/in²) of the lotion composition is applied to the topsheet using a hot melt pneumatic Dynatec E84B 1758 spray head having a head operating temperature of about 90°C and an atomization pressure of about 16 kiloPascals (kPa).

The lotion composition of Example VIII is subsequently applied to the entire wearer-contacting surface of a DRI-WEAVE topsheet of a panty liner product such as Alldays Regular manufactured by the Procter & Gamble Company. To deliver a safe and effective amount of the lotion composition onto the skin, about 0.4 mg/cm² (2.6 mg/in²) of the lotion composition is applied to the topsheet using a Meltex EP45 hot melt applicator having a head operating temperature of about 90°C.

The lotion composition of Example IX is subsequently applied by slot coating (Nordsen EP 11-12-02) striped configurations of the composition onto the wearer-contacting surface of a hydrophobic spunbond bicomponent polyethylene / polypropylene topsheet (BBA, Washougal, WA) of a sanitary pad product. To deliver a safe and effective amount of the lotion composition onto the skin, about 3.0 mg/cm² (19.5 mg/in²) of the lotion composition is applied to the topsheet.

For catamenial devices the amount of lotion add on level can be significantly higher that that used in other absorbent articles, such as diapers. For example, while not being bound by theory, it is believed that lotion can be added on at levels of 3 mg/cm², 4 mg/cm², 5 mg/cm², 6 mg/cm², 7 mg/cm², 8 mg/cm², 9 mg/cm² or 10 mg/cm². These levels refer to the area actually covered by lotion.

## Claims

1. A catamenial device, said device comprising:
(a) a topsheet having a body facing surface, wherein said topsheet has a level of hydrophobicity,
(b) a lotion composition applied to at least a portion of at least to the body facing surface of said topsheet,
(c) a backsheet joined to said topsheet; and
(d) an absorbent core disposed between said topsheet and said backsheet;
**characterized in that** said topsheet exhibits a surface having a contact angle with water of 90 degrees or greater and said lotion having a level of hydrophobicity equal or greater than that of said topsheet and said lotion composition comprising from 60% to 99.9% by weight of the carrier wherein the carrier is selected from the group consisting of petroleum-based hydrocarbons having from 4 to 32 carbon atoms, fatty alcohols having from 12 to 24 carbon atoms, lower alcohols having from about 1 to about 6 carbon atoms, low molecular weight glycols and polyols, lanolin, and mixtures thereof and wherein said carrier further comprises from 1 to 25% of said wetting agent, said wetting agent being nonionic and having an HLB value of from 1 to 7.

2. The catamenial device of Claim 1, wherein said topsheet comprises a nonwoven material.

3. The catamenial device of Claim 1, wherein said topsheet comprises a formed film material.

4. The catamenial device of Claim 1, wherein said topsheet exhibits a surface having a contact angle with water of at least 73 degrees.

5. The catamenial device of claim 1, wherein said topsheet exhibits a surface having a contact angle with water of at least 90 degrees.

6. The catamenial device of Claim 1, wherein said lotion composition exhibits an HLB value of less than or equal to 7.

7. The catamenial device of Claim 1, wherein said lotion composition exhibits an HLB value of less than or equal to 5.

8. The catamenial device of Claim 1, wherein said lotion composition exhibits an HLB value of less than or equal to 3.

9. The catamenial device of Claim 1, wherein said lotion composition is surfactant free.

10. The catamenial device of Claim 1, wherein said lotion is disposed on said topsheet non-uniformly.

11. The catamenial device of Claim 1, wherein said lotion is disposed on said topsheet in a stripe generally longitudinally oriented to said device.

12. The catamenial device of Claim 1, wherein said lotion is disposed on said topsheet in generally parallel stripes.

13. The catamenial device of Claim 1, wherein the petroleum based carrier further comprises fatty alcohols having from 12 to 24 carbon atoms, alkyl ethoxylates, fumed silica, talc, bentonites, hectorites, calcium silicates, magnesium silicates, magnesium aluminum silicates, zinc stearates, sorbitol, colloidal silicone dioxides, spermaceti, camuba wax, beeswax, candelilla wax, paraffin wax, microcrystalline wax, castrol wax, ceresin, esparto, ouricuri, rezowax, polyethylene wax. C12-C24 fatty acids, polyhydroxy fatty acid esters, polyhydroxy fatty acid amides, polymethacrylate polymers, polymethacrylate and styrene copolymers, or combinations thereof.

14. The catamenial device of Claim 1, wherein said lotion composition is surfactant free.

15. The catamenial device of Claim 1, wherein said wetting agent comprises an ethoxylated alcohol having an alkyl chain of from 8 to 22 carbon atoms and having an average degree of ethoxylation ranging from 1 to 4.

16. The catamenial device of Claim 1, wherein said wetting agent comprises an ethoxylated sorbitan ester of a C14 -C18 fatty acid having an average degree of ethoxylation of from 1 to 4.

17. The catamenial device of Claim 1, wherein said wetting agent comprises an ethoxylated amine ester of a C14 -C18 fatty acid having an average degree of ethoxylation of from 1 to 4.

18. The catamenial device of Claim 1, wherein said wetting agent comprises a polyethylene glycol ester of a C14 -C18 fatty acid having an average degree of ethoxylation of from 1 to 4.

19. The catamenial device of Claim 1, wherein the lotion composition further comprises from 0.001% to 10% by weight of a skin conditioning agent selected from the group consisting of panthenol, glycerine, and mixtures thereof.

20. The catamenial device of Claim 1, wherein the lotion composition further comprises a skin treatment active selected from the group consisting of allantoin, aluminum hydroxide gel, calamine, cysteine hydrochloride, racemic methionine, sodium bicarbonate, Vitamin C and derivatives thereof, serine protease, metalloprotease, cysteine protease, aspartyl protease, peptidase, phenylsulfonyl fluoride, lipase, diesterase, urease, amylase, elastase, nuclease, guanidinobenzoic acid and its salts and derivatives, chamomile, and mixtures thereof.

## Patentansprüche

1. Vorrichtung für die weibliche Intimhygiene, wobei die Vorrichtung Folgendes umfasst:
(a) eine Oberschicht mit einer auf den Körper gerichteten Oberfläche, wobei die Oberschicht einen Grad an Hydrophobie aufweist,
(b) eine Lotionszusammensetzung, die auf mindestens einen Teil von mindestens der auf den Körper gerichteten Oberfläche der Oberschicht aufgetragen ist,
(c) eine Unterschicht, die mit der Oberschicht verbunden ist, und
(d) einen Absorptionskern, der zwischen der Oberschicht und der Unterschicht angeordnet ist, **dadurch gekennzeichnet, dass** die Oberschicht eine Oberfläche mit einem Kontaktwinkel mit Wasser von 90 Grad oder mehr aufweist und die Lotion einen Hydrophobiegrad aufweist, der mindestens so hoch ist wie der der Oberschicht, und wobei die Lotionszusammensetzung zu 60 Gew.-% bis 99,9 Gew.-% den Träger umfasst, wobei der Träger ausgewählt ist aus der Gruppe bestehend aus Kohlenwasserstoffen auf Erdölbasis mit 4 bis 32 Kohlenstoffatomen, Fettalkoholen mit 12 bis 24 Kohlenstoffatomen, niederen Alkoholen mit ungefähr 1 bis ungefähr 6 Kohlenstoffatomen, niedermolekularen Glycolen und Polyolen, Lanolin und Mischungen davon, und wobei der Träger ferner zu 1 bis 25 % das Benetzungsmittel umfasst, wobei das Benetzungsmittel nichtionisch ist und einen HLB-Wert von 1 bis 7 aufweist.

2. Vorrichtung für die weibliche Intimhygiene nach Anspruch 1, wobei die Oberschicht ein Vliesmaterial umfasst.

3. Vorrichtung für die weibliche Intimhygiene nach Anspruch 1, wobei die Oberschicht ein geformtes Folienmaterial umfasst.

4. Vorrichtung für die weibliche Intimhygiene nach Anspruch 1, wobei die Oberschicht eine Oberfläche mit einem Kontaktwinkel mit Wasser von mindestens 75 Grad aufweist.

5. Vorrichtung für die weibliche Intimhygiene nach Anspruch 1, wobei die Oberschicht eine Oberfläche mit einem Kontaktwinkel mit Wasser von mindestens 90 Grad aufweist.

6. Vorrichtung für die weibliche Intimhygiene nach Anspruch 1, wobei die Lotionszusammensetzung einen HLB-Wert von höchstens 7 aufweist.

7. Vorrichtung für die weibliche Intimhygiene nach Anspruch 1, wobei die Lotionszusammensetzung einen HLB-Wert von höchstens 5 aufweist.

8. Vorrichtung für die weibliche Intimhygiene nach Anspruch 1, wobei die Lotionszusammensetzung einen HLB-Wert von höchstens 3 aufweist.

9. Vorrichtung für die weibliche Intimhygiene nach Anspruch 1, wobei die Lotionszusammensetzung tensidfrei ist.

10. Vorrichtung für die weibliche Intimhygiene nach Anspruch 1, wobei die Lotion ungleichmäßig auf der Oberschicht aufgebracht ist.

11. Vorrichtung für die weibliche Intimhygiene nach Anspruch 1, wobei die Lotion in einem Streifen, der allgemein in Längsrichtung zu der Vorrichtung ausgerichtet ist, auf der Oberschicht aufgebracht ist.

12. Vorrichtung für die weibliche Intimhygiene nach Anspruch 1, wobei die Lotion in allgemein parallelen Streifen auf der Oberschicht aufgebracht ist.

13. Vorrichtung für die weibliche Intimhygiene nach Anspruch 1, wobei der Träger auf Erdölbasis ferner Fettalkohole mit 12 bis 24 Kohlenstoffatomen, Alkylethoxylate, pyrogene Kieselsäure, Talk, Bentonite, Hectorite, Calciumsilicate, Magnesiumsilicate, Magnesium-Aluminiumsilicate, Zinkstearate, Sorbit, kolloidale Silikondioxide, Walrat, Carnaubawachs, Bienenwachs, Candelillawachs, Paraffinwachs, mikrokristallines Wachs, Castrolwachs, Ceresin, Esparto, Ouricuri, Rezowachs, Polyethylenwachs, C12-C24-Fettsäuren, Polyhydroxyfettsäureester, Polyhydroxyfettsäureamide, Polymethacrylatpolymere, Polymethacrylat- und Styrolcopolymere oder Kombinationen davon umfasst.

14. Vorrichtung für die weibliche Intimhygiene nach Anspruch 1, wobei die Lotionszusammensetzung tensidfrei ist.

15. Vorrichtung für die weibliche Intimhygiene nach Anspruch 1, wobei das Benetzungsmittel einen ethoxylierten Alkohol mit einer Alkylkette von 8 bis 22 Kohlenstoffatomen und mit einem durchschnittlichen Ethoxylierungsgrad im Bereich von 1 bis 4 umfasst.

16. Vorrichtung für die weibliche Intimhygiene nach Anspruch 1, wobei das Benetzungsmittel einen ethoxylierten Sorbitanester einer C14-C18-Fettsäure mit einem durchschnittlichen Ethoxylierungsgrad von 1 bis 4 umfasst.

17. Vorrichtung für die weibliche Intimhygiene nach Anspruch 1, wobei das Benetzungsmittel einen ethoxylierten Aminester einer C14-C18-Fettsäure mit einem durchschnittlichen Ethoxylierungsgrad von 1 bis 4 umfasst.

18. Vorrichtung für die weibliche Intimhygiene nach Anspruch 1, wobei das Benetzungsmittel einen Polyethylenglycolester einer C14-C18-Fettsäure mit einem durchschnittlichen Ethoxylierungsgrad von 1 bis 4 umfasst.

19. Vorrichtung für die weibliche Intimhygiene nach Anspruch 1, wobei die Lotionszusammensetzung ferner zu 0,001 Gew.-% bis 10 Gew.-% ein Hautkonditioniermittel, ausgewählt aus der Gruppe bestehend aus Panthenol, Glycerin und Mischungen davon umfasst.

20. Vorrichtung für die weibliche Intimhygiene nach Anspruch 1, wobei die Lotionszusammensetzung ferner einen Hautbehandlungswirkstoff umfasst, der ausgewählt ist aus der Gruppe bestehend aus Allantoin, Aluminiumhydroxidgel, Kieselzinkerz, Cysteinhydrochlorid, racemischem Methionin, Natriumhydrogencarbonat, Vitamin C und Derivaten davon, Serinprotease, Metalloprotease, Cysteinprotease, Aspartylprotease, Peptidase, Phenylsulfonylfluorid, Lipase, Diesterase, Urease, Amylase, Elastase, Nuclease, Guanidinobenzoesäure und deren Salzen und Derivaten, Kamille und Mischungen davon.

## Revendications

1. Dispositif cataménial, ledit dispositif comprenant :
(a) une feuille de dessus ayant une surface faisant face au corps, où ladite feuille de dessus a un niveau d'hydrophobie,
(b) une composition de lotion appliquée à au moins une partie d'au moins la surface faisant face au corps de ladite feuille de dessus,
(c) une feuille de fond jointe à ladite feuille de dessus ; et
(d) une âme absorbante disposée entre ladite feuille de dessus et ladite feuille de fond; **caractérisée en ce que** ladite feuille de dessus présente une surface ayant un angle de contact avec l'eau de 90 degrés ou plus et ladite lotion a un niveau d'hydrophobie égal ou supérieur à celui de ladite feuille de dessus ; et ladite composition de lotion constituant de 60 % à 99,9 % en poids du véhicule où le véhicule est choisi dans le groupe constitué d'hydrocarbures à base de pétrole ayant de 4 à 32 atomes de carbone, des alcools gras ayant de 12 à 24 atomes de carbone, des alcools inférieurs ayant d'environ 1 à environ 6 atomes de carbone, des glycols et polyol à faible masse moléculaire, de la lanoline, et leurs mélanges ; et dans lequel ledit véhicule comprend, en outre, de 1 à 25 % dudit agent mouillant, ledit agent mouillant étant non ionique et ayant un rapport hydro-lipophile allant de 1 à 7.

2. Dispositif cataménial selon la revendication 1, dans lequel ladite feuille de dessus comprend un matériau non tissé.

3. Dispositif cataménial selon la revendication 1, dans lequel ladite feuille de dessus comprend un matériau de film formé.

4. Dispositif cataménial selon la revendication 1, dans lequel ladite feuille de dessus présente une surface ayant un angle de contact avec l'eau d'au moins 75 degrés.

5. Dispositif cataménial selon la revendication 1, dans lequel ladite feuille de dessus présente une surface ayant un angle de contact avec l'eau d'au moins 90 degrés.

6. Dispositif cataménial selon la revendication 1, dans lequel ladite composition de lotion présente un rapport hydro-lipophile inférieur ou égal à 7.

7. Dispositif cataménial selon la revendication 1, dans lequel ladite composition de lotion présente un rapport hydro-lipophile inférieur ou égal à 5.

8. Dispositif cataménial selon la revendication 1, dans lequel ladite composition de lotion présente un rapport hydro-lipophile inférieur ou égal à 3.

9. Dispositif cataménial selon la revendication 1, dans lequel ladite composition de lotion est exempte d'agent tensioactif.

10. Dispositif cataménial selon la revendication 1, dans lequel ladite lotion est disposée non uniformément sur ladite feuille de dessus.

11. Dispositif cataménial selon la revendication 1, dans lequel ladite lotion est disposée sur ladite feuille de dessus en une bande généralement orientée longitudinalement par rapport audit dispositif.

12. Dispositif cataménial selon la revendication 1, dans lequel ladite lotion est disposée sur ladite feuille de dessus en bandes généralement parallèles.

13. Dispositif cataménial selon la revendication 1, dans lequel le véhicule à base de pétrole comprend, en outre, des alcools gras ayant de 12 à 24 atomes de carbone, des éthoxylates d'alkyle, de la silice fumée, du talc, des bentonites, des hectorites, des silicates de calcium, des silicates de magnésium, des silicates de magnésium et d'aluminium, des stéarates de zinc, du sorbitol, des dioxydes de silicium colloïdaux, du spermaceti, de la cire de carnauba, de la cire d'abeille, de la cire de candililla, de la cire de paraffine, de la cire microcristalline, de la cire castrol, de la cérésine, de la luzerne, de l'ouricuri, de la rezowax, de la cire de polyéthylène, des acides gras en C 12 à C24, des esters d'acide gras polyhydroxyle, des amides d'acide gras polyhydroxyle, des polymères polyméthacrylate, des copolymères polyméthacrylate et styrène, ou leurs combinaisons.

14. Dispositif cataménial selon la revendication 1, dans lequel ladite composition de lotion est exempte d'agent tensioactif.

15. Dispositif cataménial selon la revendication 1, dans lequel ledit agent mouillant comprend un alcool éthoxylé ayant une chaîne alkyle allant de 8 à 22 atomes de carbone et ayant un degré moyen d'éthoxylation allant de 1 à 4.

16. Dispositif cataménial selon la revendication 1, dans lequel ledit agent mouillant comprend un ester de sorbitan éthoxylé d'un acide gras en C14 à C18 ayant un degré moyen d'éthoxylation allant de 1 à 4.

17. Dispositif cataménial selon la revendication 1, dans lequel ledit agent mouillant comprend un ester d'amine éthoxylé d'un acide gras en C 14 à C18 ayant un degré moyen d'éthoxylation allant de 1 à 4.

18. Dispositif cataménial selon la revendication 1, dans lequel ledit agent mouillant comprend un ester de polyéthylène glycol d'un acide gras en C 14 à C18 ayant un degré moyen d'éthoxylation allant de 1 à 4.

19. Dispositif cataménial selon la revendication 1, dans lequel la composition de lotion comprend, en outre, de 0,001 % à 10 % en poids d'un agent de conditionnement de la peau choisi dans le groupe constitué de panthénol, glycérine, et leurs mélanges.

20. Dispositif cataménial selon la revendication 1, dans lequel la composition de lotion comprend en outre un principe actif de traitement de la peau choisi dans le groupe constitué d'allantoïne, gel d'hydroxyde d'aluminium, calamine, chlorhydrate de cystéine, méthionine racémique, bicarbonate de sodium, vitamine C et ses dérivés, sérine protéase, métalloprotéase, cystéine protéase, aspartyl protéase, peptidase, fluorure de phénylsulfonyle, lipase, diestérase, uréase, amylase, élastase, nucléase, acide guanidinobenzoïque et ses sels et dérivés, camomille, et leurs mélanges.
